(19) Europäisches Patentamt
European Patent Office
Office européen des brevets

(11) Veröffentlichungsnummer: **0 029 181 A1**

# (12) EUROPÄISCHE PATENTANMELDUNG

(21) Anmeldenummer: 80106834.7

(22) Anmeldetag: 06.11.80

(51) Int. Cl.³: **C 08 F 14/06**, C 08 F 2/16, C 23 F 15/00

(30) Priorität: 17.11.79 DE 2946461

(43) Veröffentlichungstag der Anmeldung: 27.05.81 Patentblatt 81/21

(84) Benannte Vertragsstaaten: BE CH DE FR GB IT LI NL SE

(71) Anmelder: HOECHST AKTIENGESELLSCHAFT, Zentrale Patentabteilung Postfach 80 03 20, D-6230 Frankfurt/Main 80 (DE)

(72) Erfinder: Fischer, Edgar, Dr., Assmannshäuser Weg 8, D-6000 Frankfurt am Main 71 (DE)
Erfinder: Brandrup, Johannes, Dr., Am Allersberg 6, D-6200 Wiesbaden (DE)
Erfinder: Weinlich, Jürgen, Dr., Eichenweg 1, D-6239 Eppstein/Taunus (DE)

## (54) Verfahren zur Herstellung von Vinylchlorid-Polymerisaten, Mittel zur Beschichtung von Polymerisationsgefässen sowie das damit beschichtete Polymerisationsgefäss.

(57) Die vorliegende Erfindung betrifft u.a. ein Verfahren zur Herstellung von Vinylchloridpolymerisaten durch Polymerisation des Monomeren oder Monomerengemisches in wäßriger Dispersion mit radikalbildenden Katalysatoren, gegebenenfalls Suspensionsstabilisatoren, Emulgatoren und weiteren Polymerisationshilfsstoffen, welches dadurch gekennzeichnet ist, daß die Polymerisation in einem Reaktor durchgeführt wird, dessen Innenwände und dessen übrige Teile, an denen sich Polymerablagerungen bilden können, ganz oder teilweise mit einem Überzug versehen sind, der bestimmte Verbindungen der allgemeinen Formel

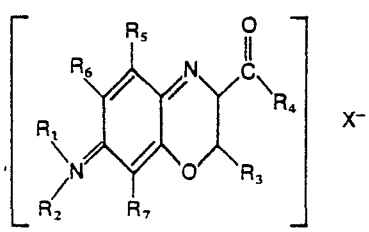

enthält.

Erfindungsgemäß läßt sich insbesondere bei Vinylchlorid-Copolymerisaten eine weitgehende Unterdrückung der Bildung von Polymerisatbelägen erreichen.

EP 0 029 181 A1

HOECHST AKTIENGESELLSCHAFT HOE 79/F 309 Dr.ZR/Wa

Verfahren zur Herstellung von Vinylchlorid-Polymerisaten, Mittel zur Beschichtung von Polymerisationsgefäßen sowie das damit beschichtete Polymerisationsgefäß

Bei der Herstellung von Vinylchlorid-Polymerisaten durch Polymerisation in wäßrigem Medium bilden sich im Verlauf der Polymerisation an der Innenwand der Polymerisationsautoklaven sowie an den Einbauten Polymerisatbeläge. Durch diese Beläge wird die Polymerisatausbeute vermindert und die Qualität des Produktes verschlechtert, da die Verkrustungen teilweise abfallen und in das Endprodukt gelangen und dort zu Stippen oder "Fischaugen" führen. Die Beläge behindern außerdem die Abfuhr der Polymerisationswärme durch die Reaktorwandungen, wodurch unwirtschaftlich lange Reaktionszeiten in Kauf genommen werden müssen.

Die Entfernung derartiger Beläge ist daher unumgänglich, was üblicherweise auf mechanischem Wege geschieht. Hierfür werden zumeist Druckwasser-Ausspritzgeräte verwendet, die jedoch nur die leicht haftenden Wandabscheidungen entfernen. Daher muß der Reaktor jeweils nach wenigen Ansätzen unter aufwendigen Sicherheitsvorkehrungen bestiegen und zusätzlich von Hand mittels Spachtel gereinigt werden. Diese Reinigungsarbeiten sind arbeitsintensiv und kostspielig, verursachen lange Stillstandzeiten und vermindern somit die Wirtschaftlichkeit des Verfahrens beträchtlich.

Es hat daher nicht an Versuchen gefehlt, derartige Polymerisationsbeläge bei der Herstellung von Vinylchloridpolymerisaten in wäßriger Dispersion zu vermindern oder gleich zu vermeiden. Eine allseits befriedigende Lösung des Problems wurde aber noch nicht erreicht.

Von den zahlreichen bekannten Verfahren bezwecken einige durch verfahrenstechnische Maßnahmen die Belagsbildung zu vermindern. Beispielsweise seien hier genannt: das Abstreifen der Wände des Reaktors mit einem entsprechend

ausgebildeten Rührer, die Einregulierung der Wandtemperatur auf mindestens die Temperatur des Reaktionsmediums, die Kühlung der Reaktorwand auf -15°C bis 0°C, das Zuführen wäßriger Lösungen, beispielsweise von Salzen der Permangansäure, Chromsäure oder Dichromsäure während der Polymerisation an der Grenzfläche zwischen Flüssigkeit und Gasphase, die Polymerisation unter Durchleiten eines elektrischen Stromes durch das flüssige Reaktionsmedium etc.

Bei anderen bekannten Verfahren werden die Komponenten des Polymerisationsrezeptes verändert und/oder der Polymerisationsflotte weitere Stoffe zugesetzt.

Weitere bekannte Verfahren verwenden zur Belagsunterdrückung Reaktoren mit besonders ausgebildeten bzw. beschichteten Innenwänden, z.B. solche mit einer Rauhtiefe der Wand von weniger als 10 µm zusammen mit wasserlöslichen, reduzierenden anorganischen Salzen und bestimmten Rührgeschwindigkeiten; oder eine unlösliche Wandbeschichtung aus einem vernetzten polymeren Material, das polare Gruppen enthält und mit einem Aldehyd als Vernetzungskomponente hergestellt wurde; oder einen Wandüberzug der überwiegend aus Polyäthylenimin besteht, das mit einem Harnstoff, Aldehyd bzw. Diisocyanat gehärtet wurde, wobei dem Polymerisationsmedium außerdem ein zweiwertiges Zinnsalz einer anorganischen Säure als Inhibitor zugesetzt werden kann. Weiterhin sind auch Wandbeschichtungen mit polyaromatischen Aminen oder mit speziellen Benzothiazol-2-on-hydrazon-Derivaten beschrieben worden.

Zu erwähnen ist hier auch die DE-OS 20 44 259, in der die Lehre gegeben wird, u.a. organische Farbstoffe als belangsunterdrückende Mittel einzusetzen. Neben einer großen Zahl anderer Vertreter werden hierbei auch ganz allgemein Oxazin-Farbstoffe erwähnt. Konkrete Vertreter dieser umfangreichen Farbstoffklasse werden aber weder in der Beschreibung noch in den Beispielen offenbart.

Aus der EU-Pa 00 00 166 war es schließlich schon bekannt, Farbstoffe mit lösliche machenden Gruppen wie -COOH- oder $SO_3H$-Gruppen als solche oder als Salze aus wässriger Lösung zum Zwecke der Belagsunterdrückung auf die Reaktorwandungen aufzubringen. In der gleichen Patentanmeldung werden hierfür auch wasserlösliche, ionische Farbstoffe mit einem heterocyclischen Ring-System vorgeschlagen.

Die bisher für die Belagsunterdrückung bei der Vinylchlorid-Polymerisation bekannten Materialien sind keineswegs voll befriedigend. So sind sie beispielsweise nicht universell einsetzbar, mit der Folge, daß sie nicht bei allen gängigen PVC-Typen, speziell solchen mit niedrigem Molekulargewicht, verwendet werden können oder daß in manchen Fällen erst die Rezepturen speziell auf diese Stoffe abgestimmt werden müssen. Eine ganze Reihe der Wirksubstanzen muß weiterhin in einem organischen Lösungsmittel eingesetzt werden, oder es müssen wegen zu geringer Effektivität Lösungen mit vergleichsweise hohem Gehalt von z.B. mehr als 0,1 % angewendet werden, um eine befriedigende Wirkung zu erzielen. Zusätzlich zeigen viele dieser Substanzen auch noch eine unerwünschte retardierende Wirkung auf die Polymerisation. Um diesen Nachteil zu vermeiden ist es nötig, nach der Wandbehandlung mit diesen Substanzen sorgfältig nachzuspülen, um eine Beeinflussung der Polymerisation durch das überschüssige Antibelagsreagenz zu verhindern. Eine sich daraus ergebende Forderung ist eine hohe Wandaffinität der jeweiligen Substanz, so daß trotz sorgfältigen Nachspülens noch genügend Wirksamkeit auf der Wand vorhanden ist.

Aufgabe der vorliegenden Erfindung war es daher, ein Verfahren zur Herstellung von Vinylchlorid-Polymerisaten bereitzustellen, welches die Nachteile des Standes der Technik nicht besitzt bzw. welches bei der kombinierten Betrachtung obiger Punkte Vorteile gegenüber den bekannten Verfahren aufweist und bei welchem insbesondere belagsverhindernde Substanzen eingesetzt werden, die eine hohe und zeitlich langandauernde belagsverhindernde Wirkung aufweisen und auch bei der Copolymerisation von Vinylchlorid genügend wirksam sind.

Diese Aufgabe wird erfindungsgemäß dadurch gelöst, daß die Polymerisation in einem Reaktor durchgeführt wird, dessen Innenteile und dessen Einbauten teilweise oder ganz mit einem Überzug versehen sind, der aus bestimmten wasserlöslichen Oxazinfarbstoffen besteht.

Die vorliegende Erfindung betrifft daher ein Verfahren zur Herstellung von Vinylchlorid-Homo-, -Co- oder Pfropfpolymerisation, die mindestens 50 Gew.-% polymerisierte Vinylchlorideinheiten enthalten, durch Polymerisation des Monomeren oder Monomerengemisches in wäßriger Dispersion mit radikalbildenden Katalysatoren, gegebenenfalls Suspensionsstabilisatoren, Emulgatoren und weiteren Polymerisationshilfsstoffen, dadurch gekennzeichnet, daß die Polymerisation in einem Reaktor durchgeführt wird, dessen Innenwände und dessen übrige Teile, an denen sich Polymerablagerungen bilden können, ganz oder teilweise mit einem Überzug versehen sind, der Verbindungen der allgemeinen Formel

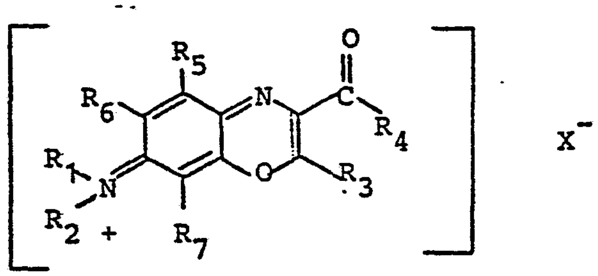

enthält, in der die einzelnen Substituenten folgendes bedeuten:

$R_1$, $R_2$ = H; gesättigter Kohlenwasserstoffrest mit 1-8 C-Atomen, vorzugsweise aliphatischer Kohlenwasserstoffrest mit 1-6 C-Atomen;

$R_3$, $R_4$ = gesättigter Kohlenwasserstoffrest mit 1-8 C-Atomen, vorzugsweise aliphatischer Kohlenwasserstoffrest mit 1-6 C-Atomen; OH; 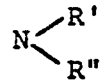 ;

R' = H oder wie übrige Reste bei $R_1/R_2$;
R" = H oder wie übrige Reste bei $R_1/R_2$; außerdem Aromat, vorzugsweise isocyclisch mit 6 bis 10 Kohlenstoffatomen, der gegebenenfalls substituiert sein kann durch eine oder mehrere der nachfolgenden Gruppen entsprechend $R_1/R_2$; O-gesättigter Kohlenwasserstoffrest mit 1 bis 8 C-Atomen, OH, COOH,

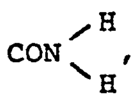, $SO_3H$, 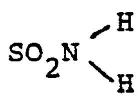; 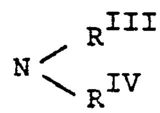

($R^{III}$, $R^{IV}$ = H oder $C_1$-$C_6$-Alkyl)

$R_4$ = zusätzlich O-gesättigter Kohlenwasserstoffrest mit 1-8 C-Atomen, vorzugsweise O-aliphatischer Kohlenwasserstoffrest mit 1-6 C-Atomen; NHOH; NH-NR'R" (R', R" wie vorstehend);

oder $R_3$ und $R_4$ = $-CH_2-CH_2-CH_2-$, 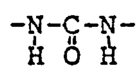;

$R_5$, $R_6$, $R_7$ = H; gesättigter Kohlenwasserstoff mit 1-8 C-Atomen, vorzugsweise aliphatischer Kohlenwasserstoffrest mit 1-6 C-Atomen; O-gesättigter Kohlenwasserstoffrest mit 1-8 C-Atomen, vorzugsweise O-aliphatischer Kohlenwasserstoffrest mit 1-6 C-Atomen; außerdem

$R_5$ und $R_6$ = isocyclischer oder heterocyclischer Aromat mit 6 bis 10 C-Atomen, gegebenenfalls substituiert mit Resten wie $R_1/R_2$

X = beliebiges einwertiges Anion oder ein entsprechendes Anionäquivalent.

Weiterhin betrifft die Erfindung ein Polymerisationsgefäß, dessen Innenwände und übrige Teile, an denen sich Polymerisationsablagerungen bilden können, ganz oder teilweise mit vorstehendem Beschichtungssystem überzogen sind.

Ein weiterer Gegenstand vorliegender Erfindung sind schließlich die Verbindungen obiger allgemeiner Formel selbst sowie belagsverhindernde Substanzen, welche obige Verbindungen enthalten.

Das Anion $X^-$ ist für die Wirksamkeit der erfindungsgemäßen Substanzen nicht entscheidend und kann daher beliebiger Natur sein. Nur beispielhaft seien hierfür genannt:
$Halogen^-$, vorzugsweise $Cl^-$ und $Br^-$, $OH^-$, $(CO_4^{--})_{1/2}$, $NO_3^-$, $(PO_4^{---})_{1/3}$, $R'''-COO^-$, ($R'''$ = $C_{1-5}$Alkyl, Aryl), $R^{IV}-SO_3$ ($R^{IV}$ = Aryl $C_{6-10}$), $\binom{COO^-}{COO^-}_{1/2}$ etc.

In obiger allgemeinen Formel bedeuten vorzugsweise:

$R_1$, $R_2$ = $C_1$-$C_6$Alkyl; wie Methyl, Äthyl, Propyl, n-Butyl, i-Butyl; n-Hexyl;

$R_3$ = OH; $C_1$-$C_6$-Alkyl, wie Methyl, Äthyl, Propyl, n-Butyl, i-Butyl; n-Hexyl; speziell Methyl;

$R_4$ = 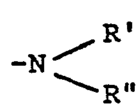 ; OH, $C_1$-$C_6$Alkyl wie Methyl, Äthyl, Propyl, n-Butyl, i-Butyl; n-Hexyl; $C_1$-$C_6$-Oxalkyl;

R' = H; $C_{1-6}$Alkyl, wie Methyl, Äthyl, Propyl, Butyl, i-Butyl,n-Hexyl;

R" = H; $C_{1-6}$Alkyl wie Methyl, Äthyl, Propyl, n-Butyl; i-Butyl;n-Hexyl; oder

$R_3$ und $R_4$ = $-CH_2-CH_2-CH_2-$

$R_5$, $R_6$, $R_7$ = H

Einige typische Vertreter von Verbindungen der obigen allgemeinen Formel sind in der nachfolgenden Tabelle I aufgeführt:

Tabelle I

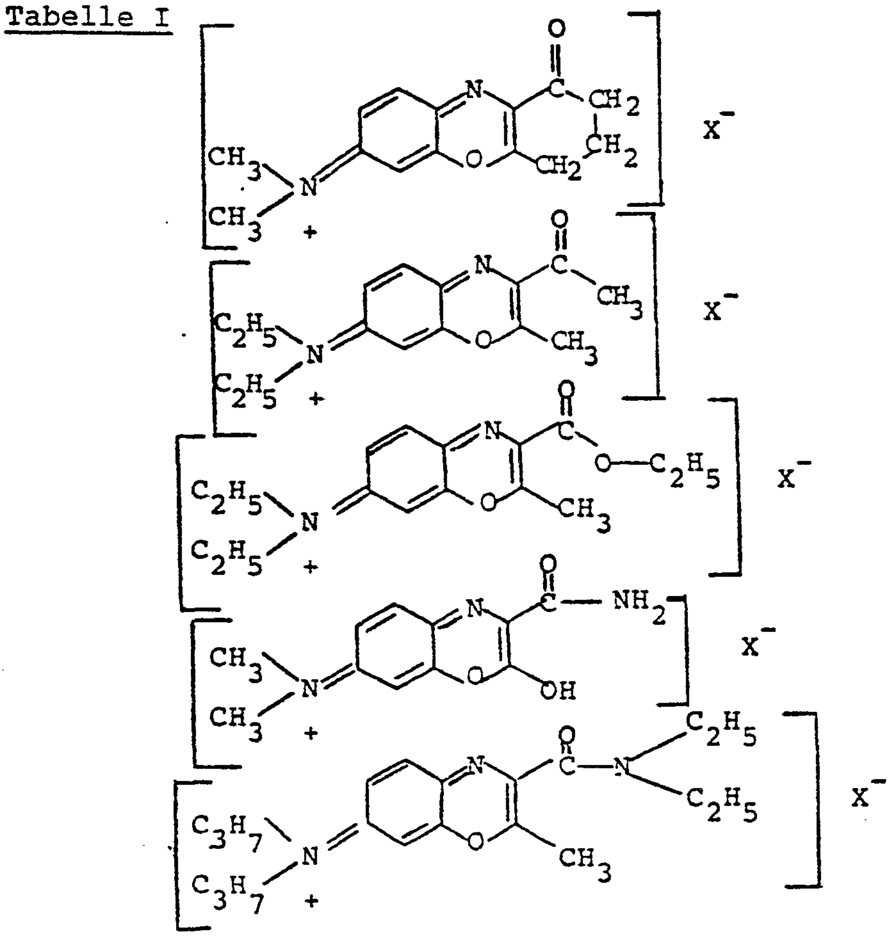

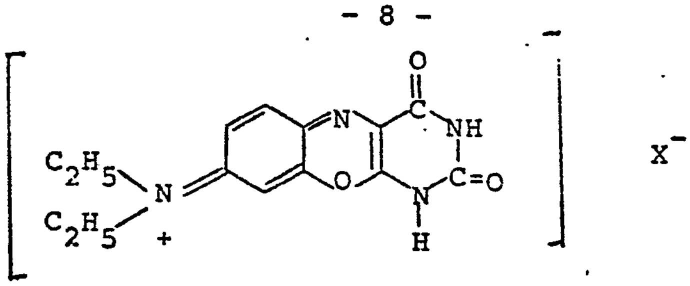

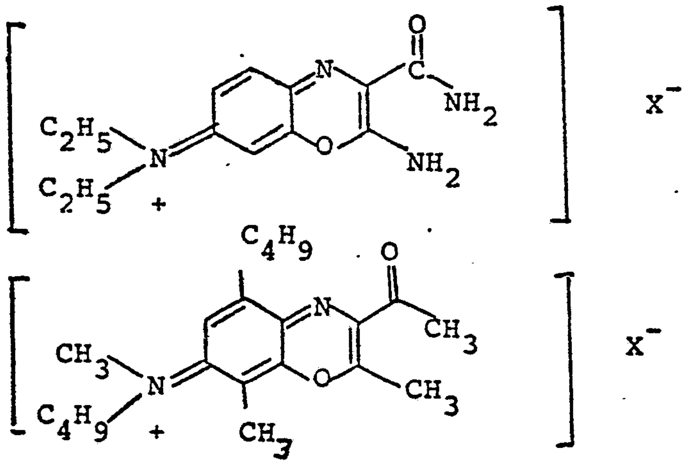

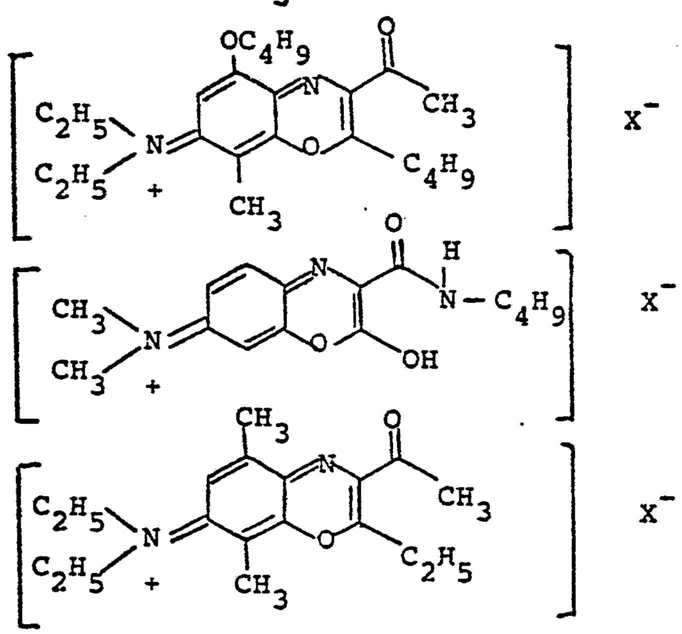

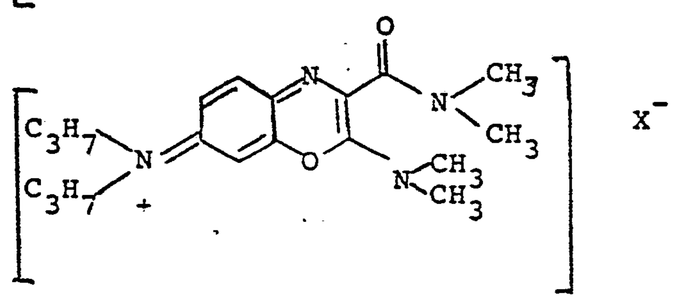

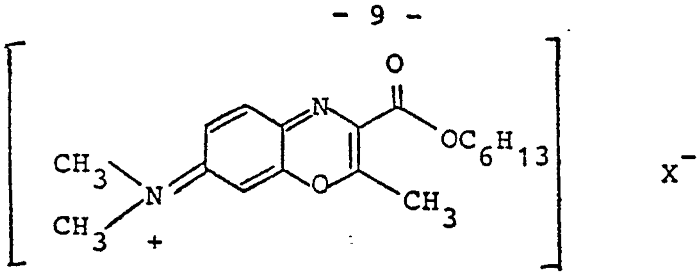

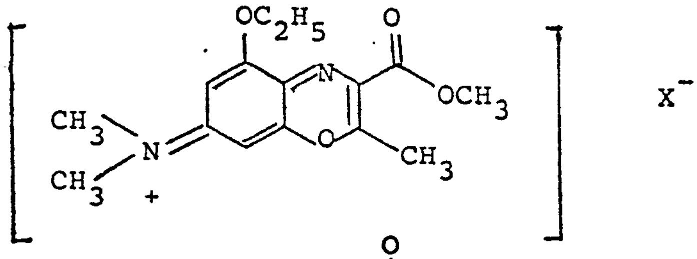

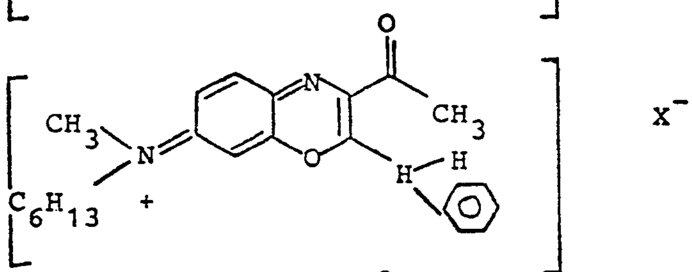

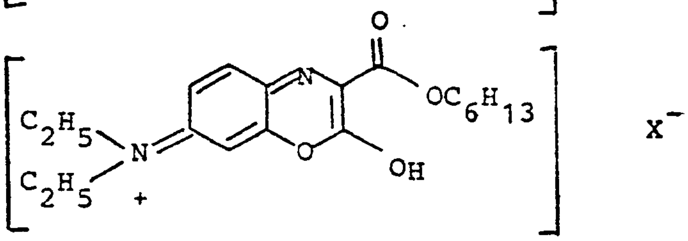

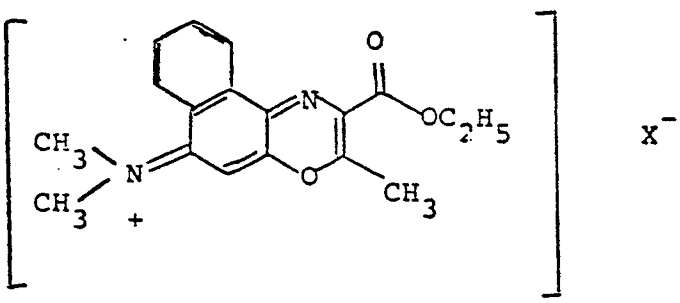

Selbstverständlich liegt es im Rahmen der Erfindung, die oben definierten Substanzen auch in Mischung untereinander einzusetzen.

Außerdem ist es möglich, die erfindungsgemäßen Substanzen mit bekannten, belagsunterdrückenden Materialien zu kombinieren, beispielsweise mit Verbindungen mit Azin- oder Thiazinringen wie Methylenblau, organischen Farbstoffen wie Nigrosinschwarz oder Anilinschwarz, anorganischen Pigmenten wie in der DE-OS 20 44 259 beschrieben oder mit polymeren Iminen gemäß der DE-OS 23 57 867 oder mit polyaromatischen Aminen wie in der DE-OS 25 41 010 offenbart. Auch die Benzothiazol-2-on-hydrazon-Derivate gemäß der DE-OS 2.703.280 und DE-OS 2.757.924 sowie die Oxazin- bzw. Thiazinfarbstoffe gemäß der DE-OS 2.919.258 bzw. DE-OS 2.919.197 kommen hierfür in Betracht.

Weiterhin können die erfindungsgemäßen Substanzen in Kombination mit Halogeniden, Hydroxiden, Oxiden und Carboxylaten irgendeines metallischen Elementes gemäß der DE-OS 25 57 788, insbesondere Zinn-II-Salzen eingesetzt werden, wobei sich zwischen der erfindungsgemäßen Substanz und dem Zusatz gegebenenfalls Komplexe in situ bilden können. Unter Umständen können derartige Metallkomplexe mit der erfindungsgemäßen Substanz auch gleich von vornherein eingesetzt werden, z.B. mit Kupfer-, Silber-, Zink-, Zinn-, Molybdän-, Eisen-, Kobalt-, Nickelionen wie in der DE-OS 25 48 424 beschrieben.

Schließlich kommen als Zusätze unter anderem auch noch Antischaummittel, Antioxidantien, Benetzungsmittel udgl. in Frage.

Die vorstehend beschriebenen Zusatzstoffe kommen vor allem dann zum Einsatz, wenn als Trägermaterial eine vernetzende Substanz oder Substanzmischung benutzt wird, da dann eine besonders wirkungsvolle Fixierung auf den Beschichtungsflächen erfolgt.

Unter dem Begriff "Überzug" sind generell solche Beschichtungen oder Filme bzw. Oberflächenbelegungen zu verstehen, die durch Inkontaktbringen einer Lösung oder Dispersion der erfindungsgemäßen Substanzen -ggf. in Kombination mit anderen bekannten belagsunterdrückenden Substanzen bzw. entsprechenden Hilfssubstanzen gemäß Seite 10- mit den jeweiligen Innenteilen des Reaktors entstehen, beispielsweise durch Besprühen, Spülen udgl., aber auch solche Beschichtungen, die unter Mitverwendung einer filmbildenden, vorzugsweise vernetzten Trägersubstanz erhältlich sind.

Die Menge an aufgebrachter Substanz gemäß obiger allgemeiner Formel beträgt in Abhängigkeit vom Polymerisationstyp, der Rezeptur, der Wandbeschaffenheit des Reaktors etc. zweckmäßigerweise mehr als 0,001 $g/m^2$ und vorzugsweise mehr als 0,01 $g/m^2$. Die Obergrenze ist in erster Linie durch wirtschaftliche Überlegungen begrenzt und liegt in der Regel bei etwa 0,1 $g/m^2$.

Da die erfindungsgemäßen Verbindungen elektronenaustauschfähig sind und durch Luftsauerstoff oder beispielsweise peroxidische Initiatoren oxidiert werden, liegen in den erhaltenen Überzügen neben der eigentlichen Verbindung gemäß obiger Formel auch deren Oxydationsprodukte vor. Es ist zu vermuten, daß die sich dabei an den beschichteten Flächen bildenden Ladungsübergangskomplexe (Charge-Transfer-Komplexe) oder Semichinonstrukturen wesentliche Wirkungsträger sind.

Die Fixierung der Verbindungen gemäß obiger allgemeiner

Formel auf den zu beschichteten Reaktorteilen kann, wie vorstehend erwähnt, in der Weise erfolgen, daß eine zusätzliche filmbildende, vorzugsweise vernetzende Trägersubstanz verwendet wird, wie in der DE-OS 27 03 280 und und DE-OS 27 57 924 beschrieben.

Vorzugsweise werden die erfindungsgemäßen Verbindungen jedoch, da sie ein genügend starkes Aufziehvermögen auf die entsprechenden Reaktorteile besitzen, ohne zusätzlichen Träger eingesetzt, d.h. die zu beschichtenden Flächen werden einfach mit einer Lösung oder Dispersion dieser Verbindung behandelt. Gegebenenfalls kann diese Behandlung nach entsprechender Zwischentrocknung und eventueller Erwärmung mehrfach wiederholt werden. In der Regel reicht jedoch eine einmalige Behandlung aus. Diese Behandlung erfolgt nach den üblichen Verfahren im allgemeinen bei Normaltemperatur, z.B. durch Spülen, Ausspülen, Einstreichen, Besprühen mit Spüldüsen, udgl. und zwar in der Regel vor jedem Polymerisationsansatz. Es hat sich dabei als zweckmäßig erwiesen, die behandelten Flächen vor der Polymerisation mit etwa der gleichen Menge Wasser oder Polymerisationsflotte nachzuspülen und die abgelaufenen Lösungen zu entfernen.

Als Lösungsmittel für die erfindungsgemäßen Verbindungen zur Herstellung der entsprechenden Behandlungslösungen kommen neben Wasser vorzugsweise solche Lösungsmittel in Frage, die in Wasser zumindestens teilweise löslich (bzw. mit diesem mischbar) sind, beispielsweise niedrige Alkohole wie Methanol, Äthanol, n(i)-Propanol, n(i)-Butanol; Ätheralkohole wie Monomethylglykoläther, Ketone wie Aceton, Ester wie Äthylacetat, Butylacetat, Dimethylformamid, Dimethylacetamid, Dimethylsulfoxyd, Acetonitril sowie entsprechende Gemische untereinander sowie mit Wasser.

Vorzugsweise werden Wasser oder dessen Gemische mit niederen Alkoholen mit 1-3 C-Atomen eingesetzt und speziell

Wasser/Methanol-Mischungen, wobei der Wassergehalt dieser Mischungen größer als 50 Gew.-%, bezogen auf die Gesamtmischung, sein kann, je nach der Löslichkeit (die natürlich von der Art der Substituenten abhängt) und der gewünschten Konzentration. Substanzen, die saure oder basische Gruppen tragen, können auch durch Salzbildung bei entsprechendem pH-Wert in wässrige oder wässrig/alkoholische Lösung überführt werden. Besonders geeignet sind solche Verbindungen, die bei einem pH-Wert von größer als 7, vorzugsweise zwischen 8 und 10,5 löslich wird. Im stark sauren Bereich ist die Wirksamkeit der Substanzen mitunter erheblich vermindert.

Der Gehalt der Gebrauchslösungen an erfindungsgemäßer Substanz kann in weiten Grenzen etwa zwischen 0,1 Mol und 100mMol pro Liter schwanken. Auch die hochverdünnten Lösungen sind wenig empfindlich gegen Oxydation durch Luftsauerstoff und erfordern kein weitgehend anärobes Arbeiten. Bevorzugt werden Lösungen mit 1-50 mMol Substanz/l; diese können unter normalen Bedingungen, also an Luft gehandhabt und angewendet werden.

Die für dieses Verfahren verwendeten Behandlungslösungen besitzen speziell im alkalischen Bereich ein ausgeprägtes Reduktionsvermögen. Partiell oxidierte Lösungen zeigen keine Beeinträchtigung ihrer Wirkung. Die darin wahrscheinlich enthaltenden Charge-Transfer-Komplexe können sogar eine Wirkungssteigerung auslösen. Daher ist es nicht nötig, die Lösungen entweder frisch zuzubereiten oder die Herstellung und Lagerung unter Stickstoff-Schutz vorzunehmen. Die beanspruchten Substanzen können auch in reduzierter Form, z.B. verküpt mit Dithionit oder Formamidinsulfinsäure eingesetzt werden; teilweise sind solche Lösungen besonders ergiebig und in ihrer Wirkung gesteigert.

Um die Netzeigenschaften der Gebrauchslösungen zu verbessern, können ihnen vorteilhaft die gleichen Dispergatoren oder Netzmittel zugesetzt werden, die auch der Flotte für

die Suspensionspolymerisation zugesetzt werden; Beispiele hierfür sind in der DE-OS 27 03 280 oder der DE-OS 27 35 770 beschrieben.

Jedes Polymerisationsgefäß für die Polymerisation von äthylenisch ungesättigten Verbindungen kann mit dem erfindungsgemäßen Überzug, gegebenenfalls bei Nichtmetallreaktoren unter Mitverwendung von Trägersubstanzen (Lacken) zur Fixierung, versehen werden. Somit können die zu beschichtenden Flächen aus den verschiedensten Materialien bestehen, z.B. aus Glas, Emaille bzw. Schmelzglasur oder Metall, vorzugsweise Stahl. Die größten Probleme bezüglich Polymerabscheidung treten im allgemeinen in Stahlreaktoren auf, so daß diese bevorzugt für den erfindungsgemäßen Überzug in Frage kommen.

Neben den Innenwänden des Polymerisationsreaktors können sich auch Polymerablagerungen an den sogenannten Einbauten, wie Rührvorrichtungen, Stromstörern (Prallblechen), Einfüllstutzen, Ventilen, Pumpen, Rohrleitungen, Meßinstrumenten und Innenkühlern (Wärmeaustauschern) bilden, die daher gleichfalls ganz oder teilweise zu beschichten bzw. zu behandeln sind. Gleiches gilt auch für Außenkühler, sofern diese mehr oder weniger direkt auf das Polymerisationsgefäß aufgesetzt sind. Gegebenenfalls kann es auch von Vorteil sein, der Polymerisationsflotte geringe Mengen der erfindungsgemäßen belagsunterdrückenden Substanz, beispielsweise 50 - 100 ppm zuzugeben.

Bei dem erfindungsgemäßen Verfahren zur Herstellung von Vinylchlorid-Polymerisation wird die Polymerisation selbst nach üblichen Methoden durchgeführt, wobei Vinylchlorid-Homopolymerisat, -Pfropfcopolymerisate oder -Copolymerisate hergestellt werden können, und zwar nach der kontinuierlichen oder diskontinuierlichen Verfahrensweise, mit oder ohne Verwendung eines Saat-Vorpolymerisates etc. Es kann dabei in wäßriger Dispersion, d.h. in Emulsion oder Suspension

/

in Gegenwart der üblichen Initiatoren, Emulgatoren bzw. Suspensionsstabilisatoren und gegebenenfalls weiteren Polymerisationshilfsstoffen polymerisiert werden. Das Polymerisationsverfahren kann unter Rückflußkühlung sowie ferner auch in der Fahrweise mit gefülltem Reaktor durchgeführt werden, bei der also das Reaktionsgefäß mit dem Polymerisationsmedium vollständig gefüllt ist und während der gesamten Polymerisation durch entsprechendes Nachdosieren in diesem Zustand gehalten wird.

Als Initiatoren, die zweckmäßigerweise in Mengen von 0,01 bis 3 Gew.-%, vorzugsweise 0,1 - 0,3 Gew.-%, bezogen auf Monomere, eingesetzt werden, seien beispielsweise genannt:

Diaryl-, Diacylperoxide, wie Diacetyl-, Acetylbenzoyl-, Dilauroyl-, Dibenzoyl-, Bis-2,4-dichlorbenzoyl-, Bis-2-Methylbenzoyl-peroxid; Dialkylperoxide wie Di-tert-butylperoxid; Perester wie tert. -Butylpercarbonat, tert.-Butylperacetat, tert.-Butylperoctat, tert.-Butylperpivalat; Dialkylperoxiddicarbonate wie Diisopropyl-, Diäthylhexyl-, Dicyclohexyl-, Diäthylcyclohexylperoxiddicarbonate; gemischte Anhydride von organischen Sulfopersäuren oder organischen Säuren wie Acetylcyclohexylsulfonylperoxid; als Polymerisationskatalysatoren bekannte Azoverbindungen, wie Azoisobuttersäurenitril; außerdem Persulfate, wie Kalium-, Natrium- oder Ammoniumpersulfate; Wasserstoffperoxid, tert.-Butylhydroperoxid oder andere wasserlösliche Peroxide, sowie auch entsprechende Gemische. Im Falle von peroxidischen Katalysatoren können diese auch in Gegenwart von 0,01 - 1 Gew.-%, bezogen auf Monomere, einer oder mehrerer reduzierender Substanzen, die zum Aufbau eines Redox-Katalysatorsystems geeignet sind, wie z.B. Sulfite, Bisulfite, Dithionite, Thiosulfate, Aldehyd-Sulfoxylate, z.B. Formaldehydsulfoxylat, eingesetzt werden. Gegebenenfalls kann die Polymerisation in Gegenwart von löslichen Metallsalzen, beispielsweise des Kupfers, Silbers, Eisens oder Chroms, durchgeführt werden, wobei die

Menge zweckmäßigerweise 0,05 - 10 ppm (auf Metallbasis, bezogen auf Monomeres) beträgt.

Ferner kann die Polymerisation, falls sie nach dem Suspensionsverfahren durchgeführt wird, in Gegenwart von 0,01 - 1 Gew.-%, vorzugsweise 0,05 - 0,3 Gew.-%, bezogen auf Monomere, von einem (oder mehreren) Schutzkolloid(en), wie beispielsweise Polyvinylalkohol, der gegebenenfalls noch bis zu 40 Mol-% Acetylgruppen enthält, Cellulosederivaten, wie wasserlösliche Methylcellulose, Carboxymethylcellulose, Hydroxyäthylcellulose, Methylhydroxypropylcellulose sowie Gelatine, Leim, Dextran, ferner Mischpolymerisaten von Maleinsäure bzw. deren Halbestern und Styrolen stattfinden.

Außerdem kann die Polymerisation in Gegenwart von 0,01 - 5 Gew.-%, bezogen auf Monomere, von einem oder mehreren Emulgatoren durchgeführt werden, wobei die Emulgatoren auch in Mischung mit den obengenannten Schutzkolloiden eingesetzt werden können. Als Emulgatoren können anionische, amphotere, kationische sowie nichtionogene verwendet werden. Als anionische Emulgatoren sind beispielsweise geeignet: Alkali-, Erdalkali-, Ammoniumsalze von Fettsäuren, wie Laurin-, Palmitin- oder Stearinsäure; von sauren Fettalkoholschwefelsäureestern; von Paraffinsulfonsäuren; von Alkylarylsulfonsäuren wie Dodecylbenzol- oder Dibutylnaphthalinsulfonsäure, von Sulfobernsteinsäuredialkylestern, sowie die Alkali- und Ammoniumsalze von epoxygruppenhaltigen Fettsäuren, wie Epoxystearinsäure; von Umsetzungsprodukten von Persäuren, z.B. Peressigsäure mit gesättigten Fettsäuren wie Ölsäure. Als amphotere bzw. kationenaktive Emulgatoren sind beispielsweise geeignet: Alkylbetaine wie Dodecylbetain, sowie Alkylpyridiniumsalze wie Laurylpyridiniumhydrochlorid; ferner Alkylammoniumsalze, wie Oxäthyldodecylammoniumchlorid. Als nichtionogene Emulgatoren kommen beispielsweise in Frage: Teilfettsäureester mehrwertiger Alkohole, wie Glycerinmonostearat, Sorbitmonolaurat, -oleat oder -palmitat; Polyoxyäthylenäther von Fettalkoholen oder aromatischen Hydroxyverbindungen; Polyoxyäthylenester von Fettsäuren sowie Polypropylenoxid-Polyäthylenoxid-Kondensationsprodukten.

Neben Katalysatoren, gegebenenfalls Schutzkolloiden und/oder Emulgatoren kann die Polymerisation in Gegenwart von Puffersubstanzen, wie beispielsweise Alkaliacetaten, Borax, Alkaliphosphaten, Alkalicarbonaten, Ammoniak oder Ammoniumsalzen von Carbonsäuren sowie von Molekülgrößen-Reglern, wie beispielsweise aliphatischen Aldehyden mit 2-4 Kohlenstoffatomen, Chlor- oder Bromkohlenwasserstoffen, wie z.B. Di- und Tri-chloräthylen, Chloroform, Bromoform, Methylenchlorid, sowie Mercaptanen durchgeführt werden.

Die Polymerisationstemperatur beträgt in der Regel 30 - 100°C, der Polymerisationsdruck 4-40 atü und der pH-Wert 3,5 - 8.

Zur Copolymerisation mit Vinylchlorid sind beispielsweise eines oder mehrere folgender Monomerer geeignet: Olefine, wie Äthylen oder Propylen; Vinylester von geradkettigen oder verzweigten Carbonsäuren mit 2-20, vorzugsweise 2-4 Kohlenstoffatomen wie Vinylacetat, -propionat, -butyrat, -1-Äthylhexoat, Vinylisotridecansäureester; Vinylhalogenide wie Vinylfluorid, Vinylidenchlorid; Vinyläther; Vinylpyridin; ungesättigte Säuren wie Malein-, Fumar-, Acryl-, Methacrylsäure und deren Mono- oder Diester mit Mono- oder Dialkoholen mit 1-10 Kohlenstoffatomen; Maleinsäureanhydrid, Maleinsäureimid, sowie deren N-Substitutionsprodukte mit aromatischen, cycloaliphatischen sowie gegebenenfalls verzweigten, aliphatischen Substituenten; Acrylnitril; Styrol.

Zur Pfropfcopolymerisation können beispielsweise elastomere Polymerisate verwendet werden, die durch Polymerisation von einem oder mehreren folgender Monomerer erhalten wurden: Diene, wie Butadien, Cyclopentadien; Olefine, wie Äthylen, Propylen; Styrol; ungesättigte Säuren, wie Acryl- oder Methacrylsäure, sowie deren Ester mit Mono- oder Dialkoholen mit 1-10 Kohlenstoffatomen, Acrylnitril; Vinylverbindungen, wie Vinylester von geradkettigen oder verzweigten Carbonsäuren mit 2-20, vorzugsweise 2-4 Kohlenstoffatomen; Vinylhalogenide, wie Vinylchlorid, Vinylidenchlorid.

Nach der Polymerisation können den als wäßrige Dispersion anfallenden Polymerisaten weitere Stoffe, zur Stabilisierung bzw. zur Verbesserung ihrer Weiterverarbeitungseigenschaften zugesetzt werden. Anschließend wird nach üblichen Aufbereitungstechniken das trockene Polymere gewonnen.

Das erfindungsgemäße Verfahren ist vorzugsweise für die Polymerisation in wäßriger Suspension mit öl-löslichen Initiatoren unter Zusatz mindestens eines Schutzkolloids (Suspensionsstabilisators) anwendbar und insbesondere für die Herstellung von Vinylchlorid-Homopolymerisaten, sowie für Vinylchlorid-Copolymerisate mit mindestens 50 Gew.-%, vorzugsweise 80-99 Gew.-% polymerisierten Vinylchlorid-Einheiten.

Die Herstellung der erfindungsgemäßen Substanzen erfolgt analog bekannten Synthesewegen, wobei jeweils die entsprechend substituierten Ausgangsprodukte einzusetzen sind. Ein allgemeiner Syntheseweg für analoge Verbindungen ist in B. 25, 1055-1067 beschrieben. Er geht aus von einer Verbindung der Formel I (siehe nachfolgendes Reaktionsschema), die mit $HNO_2$ zu einer Verbindung der Formel II

umgesetzt wird. Die Nitrosoverbindung II wird anschließend mit einer ß-Dicarbonylverbindung III zur Reaktion gebracht, was zu den erfindungsgemäßen Substanzen mit der Struktur IV führt:

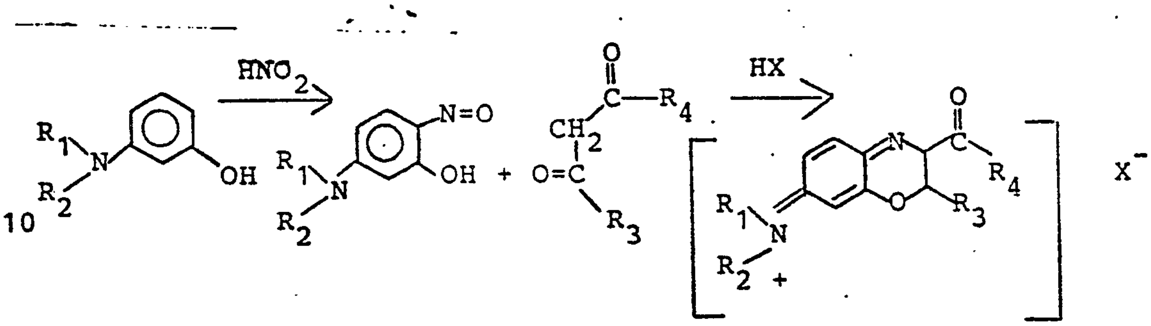


I II III IV

Die Umsetzung von I zu II erfolgt vorzugsweise in Wasser, $C_1$-$C_3$-Alkoholen, Essigsäure oder entsprechender Gemische bei Temperaturen bevorzugt unterhalb von 25°C, speziell bei 0 - 10°C. Die Reaktion von II und III zu IV wird zweckmäßigerweise in demselben Reaktionsmedium wie vorstehend beschrieben, vorzugsweise in Essigsäure, durchgeführt. Die Temperaturen liegen hier im allgemeinen bei 60 bis 150°C, vorzugsweise beim Siedepunkt des Reaktionsmediums unter Normaldruck.

Falls diese Verbindungen als Mittel zur Belagsunterdrückung eingesetzt werden ist eine Isolierung nicht unbedingt erforderlich; vielmehr können beispielsweise die erhaltenen Lösungen als solche weiter verarbeitet, d.h. verdünnt und mit weiteren Zusätzen versehen werden.

Die gute belagsunterdrückende und geringe polymerisationsinhibierende Wirkung der erfindungsgemäßen Oxazinderivate ist als überraschend anzusehen, da eine große Zahl von Vertretern der Oxazine-wie nachfolgend durch Vergleichsversuche gezeigt wird - entweder unwirksam sind oder teilweise sogar belagsfördernde Eigenschaften aufweisen und in einigen Fällen zusätzlich als Polymerisationsinhibitoren wirken.

Unerwartet und überraschend ist weiterhin, daß es beim Einsatz der beanspruchten Substanzen zu keiner Verminderung des rezepturbedingten Kornverteilungsspektrums des Polymerisates kommt, so daß eine oft langwierige und aufwendige spezielle Anpassung der Polymerisationsrezeptur an das Belagsverhinderungsverfahren nicht notwendig ist. Im Gegenteil dazu wurde sogar gefunden, daß bei Anwendung der erfindungsgemäßen Verbindungen gewisse Produkteigen-schaften deutlich im günstigen Sinne beeinflußt werden, so der für die Weichmacheraufnahme kennzeichnende ZFR-Wert (DIN 53 417, Bl. 1).

Ein weiterer Vorteil des vorliegenden Verfahrens besteht in der großen Anwendungsbreite, auch für die an sich problematischen Produkttypen mit niedrigem Molekulargewicht.

Wegen der hohen Wandaffinität der erfindungsgemäßen Oxazinderivate können diese in sehr geringen Konzentrationen eingesetzt werden, was Vorteile gegenüber den bisher bekannten Vertretern dieser Substanzklasse bringt, u.a. auch wegen der dadurch bedingten geringeren Belastung der Umwelt und der einfacheren Behandlung des Abwassers. Es wurde dabei gefunden, daß noch Konzentrationen von 0,005 % von Wirksubstanz in der Behandlungslösung einen brauchbaren Effekt zeigen, wobei man im allgemeinen jedoch Konzentrationen von 0,01 - 0,25 % bevorzugen wird.

Das erfindungsgemäße Verfahren ermöglicht es im übrigen, viele Polymerisationsansätze über lange Zeiträume ohne störende Belagsbildung an den Wänden und den Einbauten des Reaktors durchzuführen. Hierdurch wird ein konstant guter Wärmeübergang auf die Behalterwand, der durch die Überzugsschicht praktisch nicht beeinträchtigt wird, und damit gleichmäßige Produktqualität gewährleistet. Zeitaufwendige, kapazitätsmindernde Wandreinigungsarbeiten entfallen, desgleichen die sonst unumgängliche häufige Öffnung des Reaktors mit den damit verbundenen schädlichen Vinylchlorid-Emissionen. Bei der kontinuierlichen Polymerisation können die Zeiträume bis zur Abstellung des Kontinuums um ein Vielfaches verlängert werden.

Ein weiterer Vorteil des erfindungsgemäßen Verfahrens besteht darin, daß es auch in älteren Reaktoren mit erheblichen Wandrauhigkeiten, die besonders stark die Belagsbildung fördern, anwendbar ist, da durch den erfindungsgemäßen Überzug die Keimfunktion dieser Wandporen wirksam

unterdrückt wird. Dies gilt insbesondere bei Einsatz von besonders aktiven Komplexbildnern der bevorzugten Struktur mit denen sich außerdem Überzüge besonders hoher Haftfestigkeit und Dauerhaftigkeit erzielen lassen, die insbesondere auch bei der Copolymerisation von Vinylchlorid gute belagsunterdrückende Wirkung zeigen.

Die erfindungsgemäßen Verbindungen können neben ihrem Einsatz als belagsverhindernde Mittel aufgrund ihrer komplexbildenden Eigenschaften mit Vorteil auch auf dem Gebiet des Korrosionsschutzes, in der Galvanotechnik, als Depolarisatioren und für serologische Tests verwendet werden.

Die Erfindung wird nachfolgend anhand von Beispielen näher beschrieben.

Beispiele 1 bis 6 und Vergleichsversuche A bis G

I. Herstellung der erfindungsgemäßen Oxazin-Derivate

Die Herstellung wird anhand folgender Verbindung erläutert:

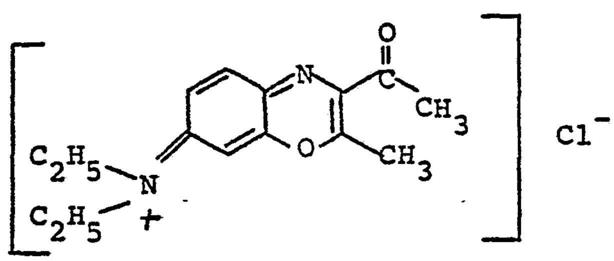

73,64 Gew.-Teile Eisessig und 5,355 Gew.-Teile Salzsäure, 37,2 Gew.-%ig wurden vorgegeben und 7,44 Gew.-Teile 3-Diäthyl-aminophenol darin bei 20°C gelöst. Dann wurde bei 15-20°C mit 7,75 Gew.-Teilen $NaNO_2$-Lösung (40 %ig) nitrosiert. Anschließend wurden 4,5 Gew.-Teile Acetylaceton eingetragen und 3 Stunden unter Rückfluß gekocht. Das Reaktionsgemisch wird im Laufe von ca. 2 Stunden auf Raumtemperatur abkühlen gelassen.

Zur Aufarbeitung wurde der Ansatz in 80 Gew.-Teile Wasser eingetragen, das ausgefallene Produkt abgesaugt, mit 150 Gew.-Teilen $H_2O$ in Portionen nachgewaschen und bei 60°C im Vakuum bis zur Gewichtskonstanz getrocknet.

Diese Synthese ist allgemein für die beanspruchten Verbindungen anwendbar, nur muß zur Vervollständigung der Umsetzung die Reaktionszeit unter Umständen von 3 auf bis zu 10 Stunden verlängert werden und die Aufarbeitung und Abscheidung von leichtlöslichen Verbindungen muß nach dem Kochen unter Rückfluß an Stelle von Wasser mit Kochsalzlösung erfolgen.

## II. Einsatz im Polymerisationsverfahren

Die sicherste Methode für eine exakte und vergleichende Ausprüfung besteht darin, absolut gleiche und gleich vorbehandelte Bleche anschließend mit den jeweiligen Substanzen zu behandeln und dann zusammen mit den zwar genau so vorbehandelten, aber ohne mit der Testsubstanz präparierten Blechen als Blindprobe in einem Reaktor zu befestigen, um die Auswertung erst nach mehreren Polymerisationsläufen vorzunehmen. Die gefundenen Ergebnisse sind dann auf die jeweils zugehörigen Blindwerte zu beziehen. In dieser Weise wurde nachfolgend verfahren:

In einem 400 l $V_4A$-Reaktor, der mit einem Impellerrührer ausgerüstet ist, wird eine $V_4A$-Manschette so gegen die Kesselwand gespannt, daß ca. 25 Stück polierte Probeplättchen mit einer Größe von 200 mm x 36 mm und einer Rauhilfe von ca. 3 µ aus kesselgleichem Material auf der Innenseite der Manschette befestigt werden können. Auf diese Probeplättchen werden die zu prüfenden Belagsverhinderungssysteme aus ihren Lösungen mit dem angegebenen Gehalt an Belagsverhinderer durch Aufpinseln aufgetragen. Bei

jeder Ausprüfungsserie werden zwei ohne Wirksubstanz behandelte Probeplättchen zur Ermittlung des Blindwertes mitgefahren.

Die Ausprüfung wird wie folgt durchgeführt:

Nach Vorlage von 215 Liter E-Wasser, welches als Dispergator gelöst 50 g teilverseiftes Polyvinylacetat und 40 g Methylhydroxypropylcellulose enthält, wird der Reaktor verschlossen, nach Verdrängen der Luft mit 115 kg Vinylchlorid chargiert und 115 g Di-2-Äthylhexylperoxydicarbonat (als 65 %ige Lösung in Aliphaten) als Aktivator nachgedrückt.

Der Reaktor wird anschließend unter Rühren (150 UpM) auf 53°C aufgeheizt und solange bei 53°C gehalten bis der Reaktordruck um 4,0 bar gefallen ist. Die Polymerisationszeit beträgt ca. 6 Stunden. Nach Reaktionsende wird das Reaktionsgemisch abgekühlt, der Polymerisationsreaktor entmonomerisiert, entleert, mit Wasser ausgespült und geöffnet.

Die Probeplättchen werden begutachtet und erneut mit Belagsverhinderern beaufschlagt. Auf diese Weise werden im Reaktor drei Ansätze durchgeführt. Nach den drei Ansätzen werden die Probeplättchen von der Manschette getrennt, getrocknet und das Gewicht des Belages der behandelten Probeplättchenfläche quantitativ bestimmt.

Die Behandlungslösungen für die Beispiele 1-6 und der Vergleichsversuche A bis G sind 0,1 %ig, bezogen auf Wirksubstanz, und bestehen aus 0,1 g Testsubstanz, 10 ml 2n NaOH und 90 ml entionisiertem Wasser.

Beim Blindwert entfällt die Testsubstanz.

Die Ergebnisse sind in der nachfolgenden Tabelle II aufgelistet und als Testwert/Blindwert angegeben. Daraus ist zu ersehen, daß viele Oxazin- und Thiazinfarbstoffe von ungenügender oder praktisch ohne Belagsverhindungswirkung sind, so die gemäß den Vergleichsversuchen B bis G. Einige davon sind sogar deutlich schlechter als der Blindwert.

Tabelle II

| Beispiel | Formel | Belag (mg) | Bemerkung |
|---|---|---|---|
| A | Blindwert | 158 (± 5 %) | Vergleichs-versuch |
| B | 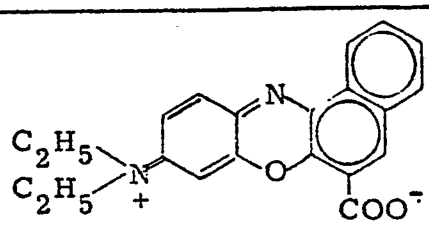 | 16o/158 | Vergleichs-versuch |
| C | 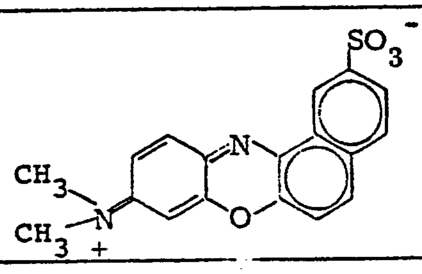 | 176/158 | Vergleichs-versuch |
| D | 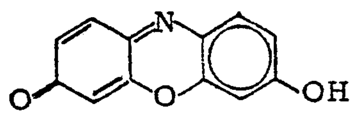 | 18o/158 | Vergleichs-versuch |
| E | 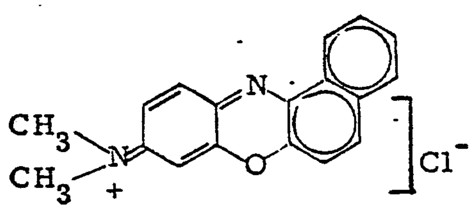 | 84/158 | Vergleichs-versuch |
| F | 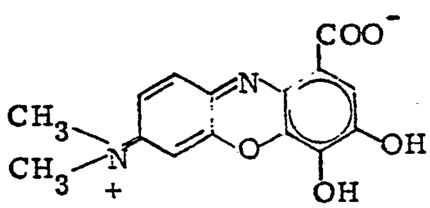 | 1o6/158 | Vergleichs-Versuch |
| G | 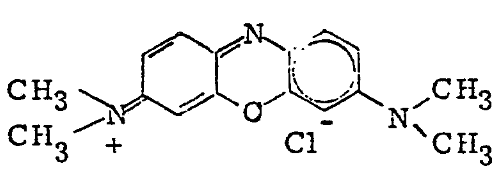 | 12o/158 | Vergleichs-versuch |

Fortsetzung Tabelle II

| Beispiel | Formel | Belag (mg) | Bemerkung |
|---|---|---|---|
| 1 | 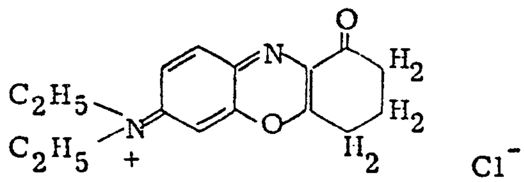 | 23/158 | erfindungsgemäß |
| 2 | 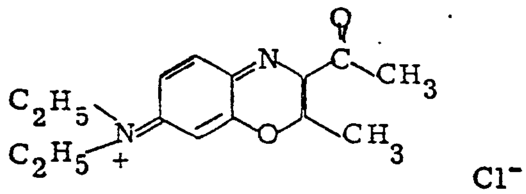 | 28/158 | erfindungsgemäß |
| 3 | 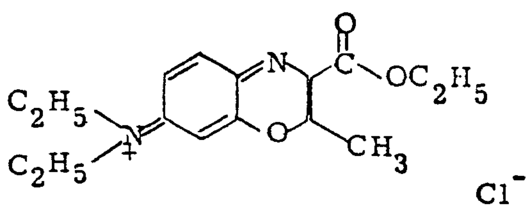 | 1o/158 | erfindungsgemäß |
| 4 | 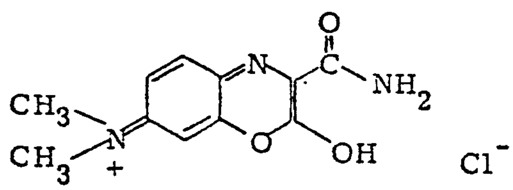 | 26/158 | erfindungsgemäß |
| 5 | 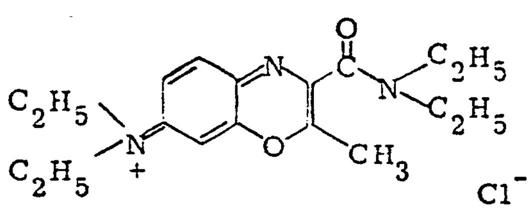 | 19/158 | erfindungsgemäß |
| 6 | 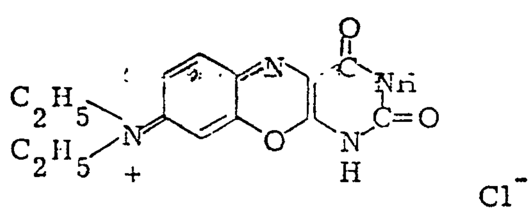 | 4o/158 | erfindungsgemäß |

Patentansprüche:

1. Verfahren zur Herstellung von Vinylchlorid-Homo-, -Co- oder -Pfropfpolymerisaten, die mindestens 50 Gew.-% polymerisierte Vinylchlorideinheiten enthalten, durch Polymerisation des Monomeren oder Monomerengemisches in wäßriger Dispersion mit radikalbildenden Katalysatoren, gegebenenfalls Suspensionsstabilisatoren, Emulgatoren und weiteren Polymerisationshilfsstoffen, dadurch gekennzeichnet, daß die Polymerisation in einem Reaktor durchgeführt wird, dessen Innenwände und dessen übrige Teile, an denen sich Polymerablagerungen bilden können, ganz oder teilweise mit einem Überzug versehen sind, die Verbindungen der allgemeinen Formel

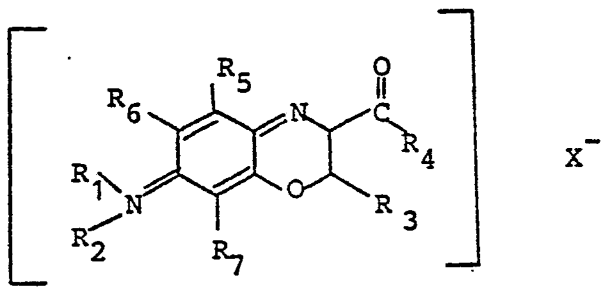

enthält, in der die einzelnen Substituenten folgendes bedeuten:

$R_1$, $R_2$ = H; gesättigter Kohlenwasserstoffrest mit 1-8 C-Atomen;

$R_3$, $R_4$ = gesättigter Kohlenwasserstoffrest mit 1-8 C-Atomen ; OH; 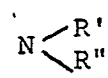 mit

R' = H oder wie übrige Reste bei $R_1/R_2$ und
R" = H oder wie übrige Reste bei $R_1/R_2$;
außerdem Aromat, vorzugsweise isocyclisch mit 6 bis 10 Kohlenstoffatomen, der gegebenenfalls substituiert sein kann durch eine oder mehrere der nachfolgenden Gruppen entsprechend $R_1/R_2$; O-gesättigter Kohlenwasserstoffrest mit 1 bis 8 C-Atomen, OH, COOH,

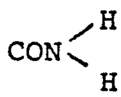 , $SO_3H$, 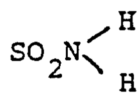 ; 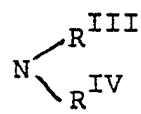

($R^{III}$, $R^{IV}$ = H oder $C_1$-$C_6$-Alkyl);

$R_4$ = zusätzlich O-gesättigter Kohlenwasserstoffrest mit 1-8 C-Atomen; NHOH; NH-NR'R" (R',R" wie vorstehend);

oder $R_3$ und $R_4$ = $-CH_2-CH_2-CH_2-$, 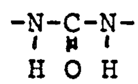 ;

$R_5$, $R_6$, $R_7$ = H; gesättigter Kohlenwasserstoffrest mit 1-8 C-Atomen; O-gesättigter Kohlenwasserstoffrest mit 1-8 C-Atomen; außerdem

$R_5$ und $R_6$ = isocyclischer oder heterocyclischer Aromat mit 6 bis 10 C-Atomen, gegebenenfalls substituiert mit Resten wie $R_1/R_2$

X = beliebiges, einwertiges Anion oder ein entsprechendes Anionäquivalent.

2. Verfahren nach Anspruch 1, dadurch gekennzeichnet, daß $R_1$ und $R_2$=$C_1$-$C_6$ Alkyl, $R_3$=$C_1$-$C_6$ Alkyl oder OH, $R_4$= $C_1$-$C_6$ Alkyl, OH oder NR'R" mit R' und R"=H oder $C_1$-$C_6$ Alkyl bedeuten oder daß $R_3$ und $R_4$= $-CH_2-CH_2-CH_2-$ sind und $R_5$ bis $R_7$ für H stehen.

3. Verfahren nach Anspruch 1 und 2, dadurch gekennzeichnet, daß die beschichteten Oberflächen vor der Polymerisation mit Wasser gespült werden.

4. Verbindungen der allgemeinen Formel

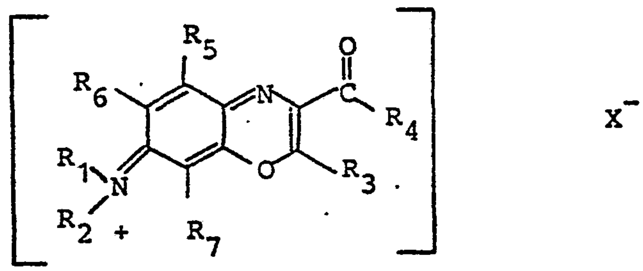

in der die Reste $R_1$ bis $R_7$ die Bedeutung gemäß Anspruch 1 und 2 haben.

5. Mittel zur Beschichtung der Innenteile von Polymerisationsgefäßen zur Unterdrückung der Wandbelagsbildung, dadurch gekennzeichnet, daß es Verbindungen gemäß Anspruch 4 enthält.

6. Polymerisationsgefäß, dadurch gekennzeichnet, daß seine Innenwände und die übrigen Teile, an denen sich Polymerablagerungen bilden können, ganz oder teilweise mit einem Überzug versehen sind, der Verbindungen der Ansprüche 1 und 2 enthält.

0029181

Europäisches Patentamt

# EUROPÄISCHER RECHERCHENBERICHT

Nummer der Anmeldung

EP 80 10 6834

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| DA | DE - A - 2 044 259 (SHIN-ETSU) | | C 08 F 14/06<br>2/16<br>C 23 F 15/00 |
| DA | EP - A - 0 000 166 (B.F. GOODRICH) | | |
| E | EP - A - 0 019 198 (HOECHST)<br>---- | | |
| | | | RECHERCHIERTE SACHGEBIETE (Int. Cl.³) |
| | | | C 08 F 14/00-<br>14/06<br>114/00-<br>114/06<br>2/00<br>C 23 F 15/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| Der vorliegende Recherchenbericht wurde für alle Patentansprüche erstellt. | | | &: Mitglied der gleichen Patentfamilie, übereinstimmendes Dokument |

| Recherchenort | Abschlußdatum der Recherche | Prüfer |
|---|---|---|
| Den Haag | 21-01-1981 | CAUWENBERG |

EPA form 1503.1. 06.78